# EUROPEAN PATENT SPECIFICATION

(11) **EP 2 428 239 B1**
(45) Date of publication and mention of the grant of the patent: **28.10.2020**
(21) Application number: 11191452.9
(22) Date of filing: 14.09.2010
(51) Int. Cl.: A61M 5/24, A61M 5/32

(54) **Hub assembly having a hidden needle for a drug delivery pen**
Hebeanordnung mit einer versteckten Nadel für einen Arzneimittelverabreichungsstift
Ensemble de moyeu doté d'une aiguille cachée pour stylo d'administration de médicaments

(30) Priority: 18.09.2009 US 563094
(43) Date of publication of application: 14.03.2012
(62) Divisional of application: 10176642.6
(73) Proprietor: Becton, Dickinson and Company, Franklin Lakes, NJ 07417-1880 (US)
(72) Inventor: Horvath, Joshua D., Sparta, New Jersey 07871 (US); Andreoni, Todd, Lyndhurst, New Jersey 07071 (US); Tuttle, Ryan M., Morristown, New Jersey 07960 (US); Gibney, Michael, Chestnut Ridge, New York 10977 (US)
(74) Representative: dompatent von Kreisler Selting Werner - Partnerschaft von Patent- und Rechtsanwälten mbB

(56) References cited:
- WO-A1-02/20074
- DE-C1- 19 856 167
- US-A- 5 015 240
- US-A- 5 658 256

## Description

### Field of the Invention

The present invention relates to a hidden needle for a pen injection device. More particularly, the present invention relates to a compressible member connected to a hub of the pen injection device that covers the needle to prevent a patient from seeing the needle, and compresses to expose the needle during the injection.

### Background of the Invention

Medication delivery pens are hypodermic syringes used for self-injection of precisely measured doses of medication. Pens are widely used, for example, by diabetics to dispense insulin.

A typical prior art medication delivery pen includes a cartridge that contains a volume of liquid medication sufficient for several doses. The dose is into a tissue area, such as the intramuscular tissue layer, the subcutaneous tissue layer, or the intradermal tissue layer.

The assembly and operation of a typical pen injection device, as shown in FIGS. 1 and 2, is described in U.S. Patent Application Publication No. 2006/0229562, published on October 12, 2006.

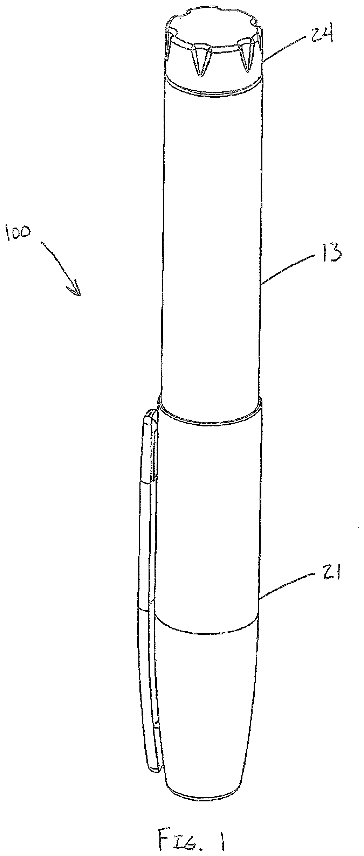

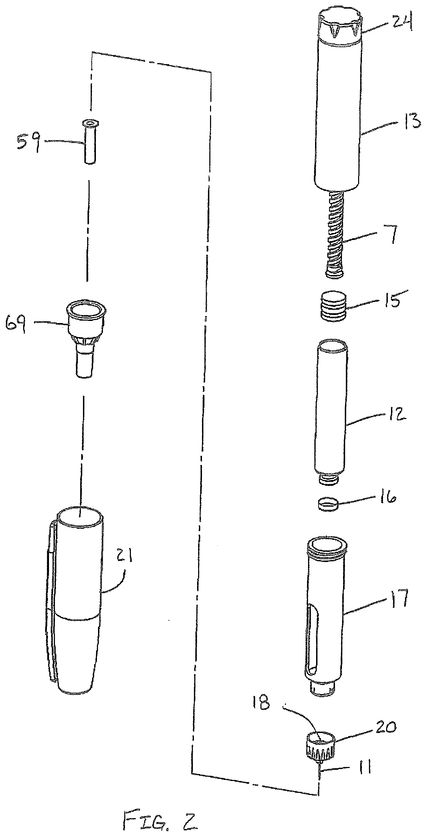

Pen injection devices, such as the exemplary pen injector 100, as shown in FIGS. 1 and 2, typically comprise a dose knob/button 24, an outer sleeve 13, and a cap 21.

The dose knob/button 24 allows a user to set the dosage of medication to be injected. The outer sleeve 13 is gripped by the user when injecting medication. The cap 21 is used by the user to securely hold the pen injector device 100 in a shirt pocket, purse or other suitable location.

FIG. 2 is an exploded view of an exemplary drug delivery pen shown in FIG. 1. The dose knob/button 24 has a dual purpose and is used to both set the dosage of the medication to be injected and to inject the dosed medicament via the lead screw 7 and stopper 15 through the medicament cartridge 12, which is attached to the drug delivery pen through a lower housing 17. In standard drug delivery pens, the dosing and delivery mechanisms are all found within the outer sleeve 13 and are not described in greater detail here as they are understood by those knowledgeable of the prior art. The distal movement of the plunger or stopper 15 within the medicament cartridge 12 causes medication to be forced into the needle 11 of the hub 20. The medicament cartridge 12 is sealed by septum 16, which is punctured by a septum penetrating needle cannula 18 located within the hub 20. The hub 20 is preferably screwed onto the lower housing 17, although other attachment means can be used such as attaching to the cartridge. To protect a user, or anyone who handles the pen injection device 100, an outer shield 69, which attaches to the hub 20, covers the hub. An inner shield 59 covers the patient needle 11 within the outer shield 69. The inner shield 59 can be secured to the hub 20 to cover the patient needle 11 by any suitable means, such as an interference fit or a snap fit. The outer shield 69 and inner shield 59 are removed prior to use. The cap 21 fits snugly against outer sleeve 13 to allow a user to securely carry the drug delivery pen 100.

The medicament cartridge 12 is typically a glass tube sealed at one end with the septum 16 and sealed at the other end with the stopper 15. The septum 16 is pierceable by a septum penetrating cannula 18, but does not move with respect to the medicament cartridge 12. The stopper 15 is axially displaceable within the medicament cartridge 12 while maintaining a fluid tight seal.

The outer shield 69 and the inner shield 59 are removed from the hub 20 and needle 11 prior to injecting a patient with the medicament stored in the cartridge 12. Some patients become uncomfortable at the sight of the needle 11, which is visible prior to the injection. Accordingly, a need exists for a pen injection device having a hub assembly that prevents a patient from seeing the needle prior to an injection.

WO 02/20074 describes a needle protection device comprising a hub and a needle cover directly connected to the hub. Upon compression of the needle cover the needle is exposed. When the needle cover is completely compressed it engages with locking means and upon expansion of the needle cover, the locking means prevents further compression of the needle cover.

US 5,015,240 describes a foldable needle cover put on a hub with a needle. The needle cover is folded back in order to expose a needle.

### Summary of the Invention

The present invention provides a hub assembly for a pen injection device in accordance to claim 1 and a method for shielding a needle from view of a pen injection device in accordance to claim 8.

In accordance with an aspect of the present invention, a hub assembly for a pen injection device has a compressible member to prevent a patient from seeing the needle prior to an injection.

In accordance with another aspect of the present invention, the compressible member allows the needle to be primed without being seen by the patient.

The hub assembly for a pen injection device according to an exemplary embodiment of the present invention prevents a user from seeing the needle prior to the injection. A hub is connected to the pen injection device. A needle is received by the hub. A compressible member is connected to the hub and is movable between a first position that entirely covers the needle and a second position that exposes the needle for an injection.

Objects, advantages, and salient features of the invention will become apparent from the following detailed description, which, taken in conjunction with the annexed drawings, discloses exemplary embodiments of the invention.

### Brief Description of the Drawings

The above benefits and other advantages of the various embodiments of the present invention will be more apparent from the following detailed description of exemplary embodiments of the present invention and from the accompanying figures, in which:
FIG. 1 is a perspective view of an assembled existing pen needle assembly;
FIG. 2 is an exploded perspective view of the components of the pen needle assembly of FIG. 1;
FIG. 3 is a perspective view of a hub assembly according to an exemplary embodiment of the present invention having a compressible member in a position covering a needle;
FIG. 4 is a perspective view of the hub assembly of FIG. 3 in which the compressible member is compressed to expose the needle;
FIG. 5 is a side elevational view of a hub assembly according to another exemplary embodiment of the present invention having a compressible member in a position covering a needle; and
FIG. 6 is a side elevational view of the hub assembly of FIG. 5 in which the compressible member is compressed to expose the needle.

Throughout the drawings, like reference numbers will be understood to refer to like parts, components and structures.

### Detailed Description of the Exemplary Embodiments

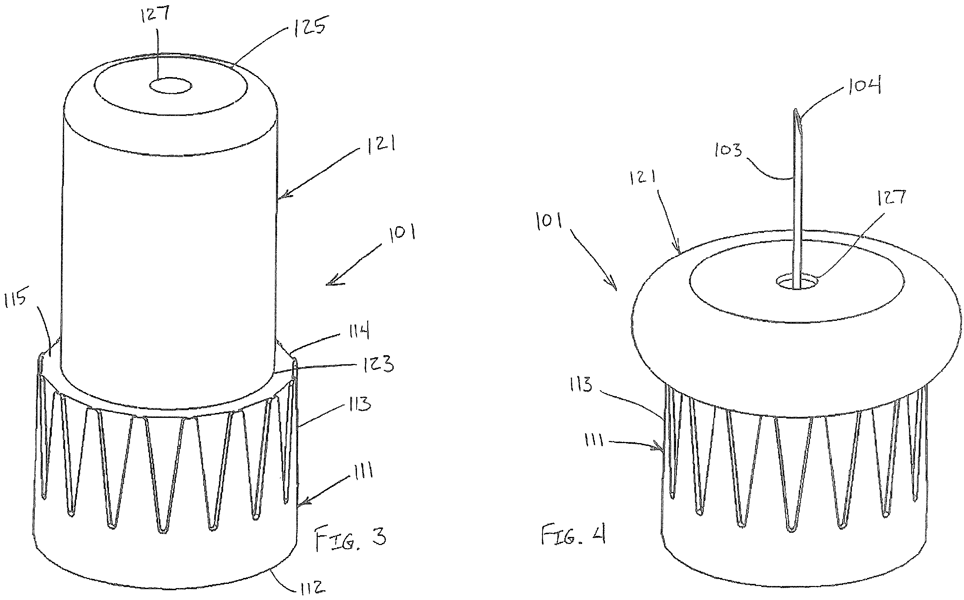

In an exemplary embodiment of the present invention, as shown in FIGS. 3 and 4, a hub assembly 101 for a pen injection device 100 (FIG. 2) prevents a patient from seeing a needle 103 of the hub assembly during the injection. A hub 111 of the hub assembly 101 is connected to the pen injection device. The needle 103 is received by the hub 111. A compressible member 121 is connected to the hub 111 such that the needle 103 is not visible during the injection.

The hub 111 is a typical hub, such as is shown in FIG. 2. The hub 111 has a body 113, which preferably has a cylindrical shape, having a first end 112 and a second end 114. A substantially planar base 115 is formed at the second end 114 of the hub body 113.

The compressible member 121 is disposed on the hub 111. An adhesive secures the first end 123 of the compressible member to the base 115 of the hub 111. The compressible member 121 has a preferably substantially cylindrical shape. An opening 127 at a second end 125 of the compressible member 121 allows the needle 103 to pass therethrough during an injection. Preferably, the compressible member 121 is a sponge made of a soft foam material.

The compressible member 121 is compressible between a first position, as shown in FIG. 3, in which the compressible member 121 is not compressed and covers substantially the entirety of the needle 103 and a second position, as shown in FIG. 4, in which the compressible member is compressed to expose the patient end 104 of the needle. Applying downward pressure on the pen injection device 100 (FIGS. 1 and 2) during an injection causes the compressible member 121 to compress, as shown in FIG. 4. Accordingly, the patient end 104 of the needle 103 is exposed such that medicament can be administered to the patient. After the medicament has been administered, the needle is removed such that pressure is no longer being applied to the compressible member.

Accordingly, the compressible member 121 returns to the non-compressed position, as shown in FIG. 3.

The compressible member 121 includes a chemical indicator that changes the color of the compressible member when the needle 103 is primed. The medicament interacts with the chemical indicator, thereby causing a color change in the compressible member 121 to indicate priming of the needle. Accordingly, the needle 103 can be primed without the needle being seen by the patient.

The compressible member 121 includes adhesive microspheres. When the compressible member 121 is compressed, as shown in FIG. 4, the adhesive microspheres rupture. When the compressible member 121 moves back to the initial position, as shown in FIG. 3, the adhesive microspheres cure, thereby hardening the compressible member so that it is no longer compressible.

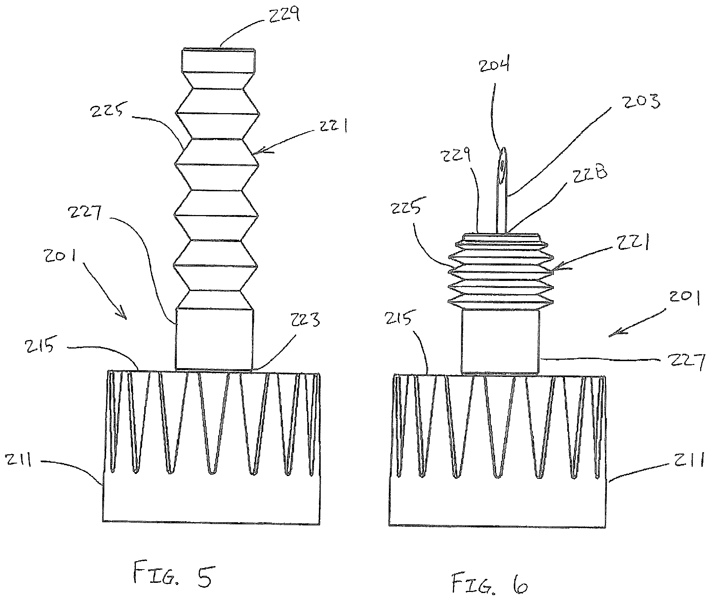

Another exemplary embodiment of a hub assembly 201 of the present invention is shown in FIGS. 5 and 6. The compressible member 221 is disposed on the hub 211. Preferably, a friction fit between the hub post and the compressible 221 member secures the compressible member to the hub 211. Alternatively, an adhesive may be used to secure a first end 223 of the compressible member 221 to the base 215 of the hub 211. The compressible member 221 has a compressible bellows portion 225 and a non-compressible portion 227. An opening 228 at a second end 229 of the compressible member 221 allows the needle 203 to pass therethrough during an injection.

The compressible member 221 is movable between a first position, as shown in FIG. 5, in which the compressible member 221 is not compressed and covers substantially the entirety of the needle 203 and a second position, as shown in FIG. 6, in which the compressible member is compressed to expose the patient end 204 of the needle. Applying downward pressure on the pen injection device 100 (FIGS. 1 and 2) during an injection causes the bellows portion 225 of the compressible member 221 to compress, as shown in FIG. 5. Accordingly, the patient end 204 of the needle 203 is exposed such that medicament can be administered to the patient. After the medicament has been administered, the needle is removed such that pressure is no longer being applied to the compressible member 221. Accordingly, the bellows portion 225 of the compressible member 221 returns to the non-compressed position, as shown in FIG. 5.

The foregoing embodiments and advantages are merely exemplary and are not to be construed as limiting the scope of the present invention. The description of exemplary embodiments of the present invention is intended to be illustrative, and not to limit the scope of the present invention. Various modifications, alternatives and variations will be apparent to those of ordinary skill in the art, and are intended to fall within the scope of the invention as defined in the appended claims and their equivalents.

## Claims

1. A hub (211) assembly for a pen injection device, comprising:
a hub (211) for connecting to the pen injection device;
a needle (203) received by said hub (211); and
a compressible member (221) connected to said hub (211) and compressible between a first position that entirely covers said needle and a second position that exposes the needle (203) for an injection,
whereby said compressible member (221) has a compressible portion (225), wherein said compressible member (221) has a non-compressible portion (227) secured to said hub (211),
**characterized in that**
the compressible member (221) comprises adhesive microspheres, whereby the adhesive microspheres rupture when the compressible member (221) is compressed such that the compressible member (221) hardens to prevent reuse when returned to a non-compressed position,
or
the compressible member (221) when contacted by medicament changes color to indicate priming of the needle (203).

2. The hub assembly of claim 1, wherein an adhesive secures said compressible member (221) to said hub (211).

3. The hub assembly of claim 1, wherein said compressible portion (221) comprises a bellows portion (225).

4. The hub assembly of claim 3, wherein the bellow portion (225) is opaque and/ or made of plastic.

5. The hub assembly of claim 1, wherein said compressible member (221) has an outer diameter smaller than an outer diameter of said hub (211) when said compressible member (221) is in said first position and/or said compressible member (221) has an opening (228) extending from a first end (223) to a second end (229) of said compressible member (221) through which said needle (203) passes during the injection.

6. A method of shielding from view of a needle of a pen injection device, comprising the steps of:
entirely covering a needle (203) received by a hub (211) of the pen injection device with a compressible member (221) connected to said hub (211);
compressing the compressible member (221) to expose the needle (203) for an injection,
rupturing adhesive microspheres in the compressible member (221) when the compressible member (221) is compressed such that the compressible member (221) hardens to prevent reuse when returned to a non-compressed position,
or
changing color of the compressible member (221) when contacted by medicament to indicate priming of the needle (203).

7. A method of shielding a needle from view of a pen injection device of claim 6, wherein, the needle (203) is primed without the needle (203) being seen by a patient.

8. A method of shielding a needle from view of a pen injection device of claim 6, further comprising uncompressing the compressible member (221) to return the compressible member (221) to a non-compressed position to recover the needle (203).

## Patentansprüche

1. Nadelansatz- (211) Anordnung für eine Pen-Injektionsvorrichtung, die aufweist:
einen Nadelansatz (211) zum Verbinden mit der Pen-Injektionsvorrichtung;
eine Nadel (203), die von dem Nadelansatz (211) aufgenommen ist; und
ein zusammendrückbares Element (221), das mit dem Nadelansatz (211) verbunden ist und zwischen einer ersten Position, in der die Nadel vollständig abgedeckt ist, und einer zweiten Position, in der die Nadel (203) für eine Injektion freiliegt, zusammendrückbar ist,
wobei das zusammendrückbare Element (221) einen zusammendrückbaren Abschnitt (225) aufweist, wobei das zusammendrückbare Element (221) einen nichtzusammendrückbaren Abschnitt (227) aufweist, der an dem Nadelansatz (211) gesichert ist,
**dadurch gekennzeichnet, dass**
das zusammendrückbare Element (221) haftfähige Mikrokugeln aufweist, wobei die haftfähigen Mikrokugeln aufreißen, wenn das zusammendrückbare Element (221) zusammengedrückt wird, so dass das zusammendrückbare Element (221) härtet, um eine Wiederverwendung zu verhindern, wenn es in eine nichtzusammengedrückte Position zurückgeführt wird, oder
das zusammendrückbare Element (221) bei Inkontaktkommen mit einem Medikament die Farbe ändert, um eine Betätigungsbereitmachung der Nadel (203) anzuzeigen.

2. Nadelansatzanordnung nach Anspruch 1, bei der ein Haftvermittler das zusammendrückbare Element (221) an dem Nadelansatz (211) sichert.

3. Nadelansatzanordnung nach Anspruch 1, bei der der zusammendrückbare Abschnitt (221) einen Faltenbalgabschnitt (225) aufweist.

4. Nadelansatzanordnung nach Anspruch 3, bei der der Faltenbalgabschnitt (225) opak und/oder aus Kunststoff gefertigt ist.

5. Nadelansatzanordnung nach Anspruch 1, bei der das zusammendrückbare Element (221) einen Außendurchmesser hat, der kleiner ist als ein Außendurchmesser des Nadelansatzes (211), wenn das zusammendrückbare Element (221) in der ersten Position ist und/oder das zusammendrückbare Element (221) eine Öffnung (228) aufweist, die sich von einem ersten Ende (223) zu einem zweiten Ende (229) des zusammendrückbaren Elements (221), durch das die Nadel (203) bei der Injektion verläuft, erstreckt.

6. Verfahren zum Abschirmen einer Nadel einer Pen-Injektionsvorrichtung gegen Blicke, das die folgenden Schritte umfasst:
vollständiges Abdecken einer Nadel (203), die von einem Nadelansatz (211) der Pen-Injektionsvorrichtung aufgenommen ist, wobei ein zusammendrückbares Element mit dem Nadelansatz (211) verbunden ist;
Zusammendrücken des zusammendrückbaren Elements (221) zum Freilegen der Nadel (203) für eine Injektion,
Aufreißen von haftfähigen Mikrokugeln in dem zusammendrückbaren Element (221), wenn das zusammendrückbare Element (221) zusammengedrückt ist, so dass das zusammendrückbare Element (221) härtet, um eine Wiederverwendung zu verhindern, wenn es in eine nichtzusammengedrückte Position zurückgeführt wird, oder
Verändern der Farbe des zusammendrückbaren Elements (221) bei Inkontaktkommen mit dem Medikament zum Anzeigen der Betätigungsbereitmachung der Nadel (203).

7. Verfahren zum Abschirmen einer Nadel einer Pen-Injektionsvorrichtung gegen Blicke nach Anspruch 6, bei dem die Nadel (203) betätigungsbereit gemacht wird, ohne dass ein Patient die Nadel (203) sieht.

8. Verfahren zum Abschirmen einer Nadel einer Pen-Injektionsvorrichtung gegen Blicke nach Anspruch 6, das ferner das Auseinanderziehen des zusammendrückbaren Elements (221) zum Zurückführen des zusammendrückbaren Elements (221) in eine nichtzusammengedrückte Position zum Einziehen der Nadel (203) umfasst.

## Revendications

1. Ensemble de raccord (211) pour un stylo injecteur, comprenant :
un raccord (211) pour une liaison au stylo injecteur ;
une aiguille (203) reçue par ledit raccord (211) ; et
un élément compressible (221) relié audit raccord (211) et pouvant être comprimé entre une première position qui couvre entièrement ladite aiguille et une deuxième position qui expose l'aiguille (203) pour une injection,
moyennant quoi ledit élément compressible (221) a une partie compressible (225), où ledit élément compressible (221) a une partie non compressible (227) fixée audit raccord (211),
**caractérisé en ce que**
l'élément compressible (221) comprend des microsphères adhésives, moyennant quoi les microsphères adhésives se rompent lorsque l'élément compressible (221) est comprimé de sorte que l'élément compressible (221) durcisse pour empêcher une réutilisation lorsqu'il est ramené à une position non comprimée, ou
l'élément compressible (221) lorsqu'il est en contact avec le médicament change de couleur pour indiquer l'amorçage de l'aiguille (203).

2. Ensemble de raccord de la revendication 1, dans lequel un adhésif fixe ledit élément compressible (221) audit raccord (211).

3. Ensemble de raccord de la revendication 1, dans lequel ladite partie compressible (221) comprend une partie soufflet (225).

4. Ensemble de raccord de la revendication 3, dans lequel la partie soufflet (225) est opaque et/ou réalisée en matière plastique.

5. Ensemble de raccord de la revendication 1, dans lequel ledit élément compressible (221) présente un diamètre externe inférieur à un diamètre externe dudit raccord (211) lorsque ledit élément compressible (221) est dans ladite première position et/ou ledit élément compressible (221) comprend une ouverture (228) s'étendant depuis une première extrémité (223) à une deuxième extrémité (229) dudit élément compressible (221) à travers laquelle ladite aiguille (203) passe pendant l'injection.

6. Procédé pour cacher une aiguille d'un stylo injecteur, comprenant les étapes consistant :
à couvrir entièrement une aiguille (203) reçue par un raccord (211) du stylo injecteur avec un élément compressible (221) relié audit raccord (211) ;
à comprimer l'élément compressible (221) pour exposer l'aiguille (203) pour une injection,
à rompre des microsphères adhésives dans l'élément compressible (221) lorsque l'élément compressible (221) est comprimé de sorte que l'élément compressible (221) durcisse pour empêcher la réutilisation lorsqu'il est ramené à une position non comprimée, ou
à changer la couleur de l'élément compressible (221) lorsqu'il est en contact avec le médicament pour indiquer l'amorçage de l'aiguille (203).

7. Procédé pour cacher une aiguille d'un stylo injecteur de la revendication 6, dans lequel l'aiguille (203) est amorcée sans que l'aiguille (203) ne soit vue par un patient.

8. Procédé pour cacher une aiguille d'un stylo injecteur de la revendication 6, comprenant en outre la décompression de l'élément compressible (221) pour ramener l'élément compressible (221) à une position non comprimée pour recouvrir l'aiguille (203).
